Europäisches Patentamt

European Patent Office

Office européen des brevets

(19)

(11) Publication number: **0 527 814 B1**

# EUROPEAN PATENT SPECIFICATION

(45) Date of publication of patent specification: **28.12.94**  (51) Int. Cl.⁵: **C12N 5/10**, C12P 21/02

(21) Application number: **91908448.3**

(22) Date of filing: **02.04.91**

(86) International application number:
**PCT/US91/02296**

(87) International publication number:
**WO 91/15573 (17.10.91 91/24)**

(54) **AN IMMORTALIZED PRIMATE HEPATOCYTE CELL LINE.**

(30) Priority: **03.04.90 US 504171**

(43) Date of publication of application:
**24.02.93 Bulletin 93/08**

(45) Publication of the grant of the patent:
**28.12.94 Bulletin 94/52**

(84) Designated Contracting States:
**AT BE CH DE DK ES FR GB GR IT LI LU NL SE**

(56) References cited:
**US-A- 7 284 368**

**Molecular and Cellular Biology, vol. 8, no. 10, October 1988, American Society for Microbiology, C.D. Woodworth et al. :"Tumorigenicity of simian virus.." pages 4492-4501, whole article.**

**Proceedings of the National Academy of Sciences, USA, vol. 81, October 1984, H.C. Isom et al..:"Ouantitative essay for albumin producing liver cells..", pages 6378-6382. Whole article.**

(73) Proprietor: **SOUTHWEST FOUNDATION FOR BIOMEDICAL RESEARCH**
**7620 N.W. Loop 410 at Military Drive,**
**P.O. Box 28147**
**San Antonio, TX 78284 (US)**

(72) Inventor: **BURK, Kenneth, H.**
**15630 Cloud Top**
**San Antonio, TX 78248 (US)**
Inventor: **JACOB, James, R.**
**5 Kinney Lane**
**Cortland NY 13045-3319 (US)**
Inventor: **LANFORD, Robert, E.**
**1902 Hillingway**
**San Antonio, TX 78248 (US)**

(74) Representative: **West, Alan Harry et al**
**R.G.C. Jenkins & Co.**
**26 Caxton Street**
**London SW1H 0RJ (GB)**

**Description**

1. Field of the Invention

The present invention relates to an immortalized primate hepatocyte cell line, and to uses of the cell line for culturing hepatotropic viruses and for producing hepatocyte secretory products.

2. References

Bishop, J.M., Cell, 42:23 (1985).
Chomozynski et al, Anal.Biochem. 162:159 (1987).
Eichberg, J. Med. Primatol. 14:165-168 (1985). Innis, M.A., et al, eds., PCR Protocols: A Guide to Methods and Applications, Academic Press (199O).
Felgner, P.L., et al, PNAS, 84:7413 (1987)
Jacob et al., in "Viral Hepatitis and Liver Diseases" Proceedings of the International Meeting on Viral Hepatitis and Liver Disease (1991, in press).
Jacob et al., Hepatology 10:921-927 (1989).
Jacob, et al., J Infect Dis, 161:1121-1127 (1990).
Jat, et al., Mol. Cell Biol. 6:1204-1217 (1986)).
Lanford et al., Virology 97:295-306 (1979).
Lanford et al., In Vitro Cell Dev. Bio., 25:174-182 (1989).
Laemmli, U.K., Nature, 227:680 (1970).
Miller, A.D., Biotechniques 7:980 (1989).
Sambrook, et al. eds. Molecular Cloning. A Laboratory Manual, Vols. 1, 2, and 3, 2nd ed. Cold Spring Harbor Laboratory Press, Cold Spring Harbor, New York (1989);
Van de Woude, G.F., et al., eds, Cancer Cells 2: Oncogenes and Viral Genes, Cold Spring Harbor Symposium, Cold Spring Harbor, NY, p 481-486.

3. Background of the Invention

Hepatocytes which can be maintained successfully in culture, in a differentiated, infectable state, would be advantageous in studying and producing human hepatotropic viruses, such as hepatitis A virus (HAV), hepatitis B virus (HVB), enterically transmitted Non-A, Non-B hepatitis virus, also now known as hepatitis E virus (HEV), and parenterally transmitted Non-A, Non-B hepatitis virus, also now known as hepatitis C virus (HCV).

Such hepatocytes would allow screening of drug compounds, for effectiveness against virus growth. The cells could also provide a source of intact, active or attenuated virus particles, for direct production of the virus, or for use in obtaining mature virus proteins or peptide fragments from isolated virus particles.

Recently, the inventors have described cell culture conditions which permit long-term growth of primary primate hepatocytes in culture, in a differentiated state (Lanford, 1989). The cultured, primary hepatocytes produce several liver-specific secretory proteins, such as albumin, $\alpha$-1-antitrypsin, complement C'4, fibrinogen, apolipoproteins A-1 and E, transferrin, and/or plasminogen for culture periods of 100 days or more. These cells are infectable by HCV and are able to support replication of the virus in culture. The use of the cultured cells to produce intact mature HCV virus particles is reported in the companion PCT patent application "Purified Non-A, Non-B Hepatitis Virus".

One limitation of primary, cultured primate hepatocytes, however, is that the differentiated cells lose many of their liver-specific functions within 3-4 weeks of infection. Therefore, new primate hepatocytes must be continually obtained, prepared for cell culture, and infected with virus when growing hepatotropic virus in culture. Another limitation of the cells is the problem of maintaining controlled, uniform cell properties for virus growth when the hepatocytes are continually being replaced by new cells.

The published US-A 7 284 368 allegedly discloses immortalized primate hepatocyte cell lines which integrate the oncogene SV-40 large T-antigen gene, which secrete albumin and are capable of supporting the replication of human hepatotropic viruses.

This document however does not provide any evidence that the described cell lines are actually immortalized, that they actually maintain prolonged hepatocyte function and that they actually exhibit the ability to support hepatotropic virus replication.

4. Summary of the Invention

One general object of the invention is to provide an immortalized primate hepatocyte cell line able to support the growth of hepatotropic viruses over extended culture periods.

The invention includes, in one aspect, an immortalized primate hepatocyte cell line whose cells are characterized by (a) an oncogene integrated in the cellular genome; (b) synthesis and secretion of normal liver secretory proteins, and (c) ability to support replication of human hepatotropic viruses. More specifically, the cells are characterized by stable secretion in culture of several, i.e., at least three of the following liver secretory products: albumin, α-1-antitrypsin, complement C'4, fibrinogen, apolipoproteins A-1 and E, transferrin, and plasminogen.

In one embodiment, the cell line is derived from chimpanzee or human hepatocytes, and the cells are infectable with and support replication of hepatitis C virus. The oncogene in one preferred immortalized chimpanzee and marmoset hepatocyte cell line is the SV40 large T antigen.

In another embodiment, the cell line is derived from baboon hepatocytes, and the oncogene is a combination of adenovirus E1A and cellular myc oncogenes.

The invention also includes a method of producing hepatotropic virus particles. The immortalized primate hepatocyte cell line, infected with the virus, is cultured under conditions which maintain the liver-specific functions of the cell, and virus particles produced by the cells are harvested from the culture medium.

In one preferred embodiment, the cell line is derived from human or chimpanzee hepatocytes, and the virus is hepatitis C virus (HCV).

Also disclosed is a method for screening compounds for the ability to inhibit growth of an hepatotropic virus. In this method, the immortalized primate hepatocyte cell line from above is cultured under conditions which maintain the liver-specific functions of the cell. After exposing the cell to virus, cultured cells are exposed to a test compound for a selected period of time, and the cells are assayed for the amount of virus present, typically by assaying for the presence of viral nucleic acid.

In still another embodiment, the invention includes a method of producing liver secretory proteins, such as albumin, α-1-antitrypsin, complement C'4, fibrinogen, apolipoproteins A-1 and E, transferrin, and plasminogen, by isolating the desired protein from the above immortalized cells in culture.

These and other objects and features of the present invention will become more readily apparent when the following detailed description of the invention is read in conjunction with the accompanying drawings.

Brief Description of the Drawings

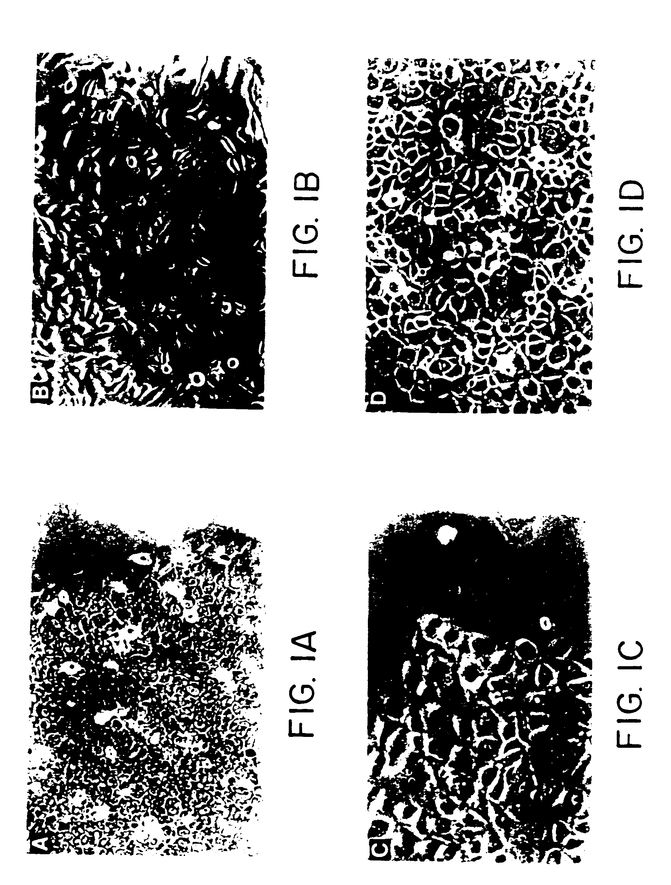

Figures 1A-1D are phase contrast photomicrographs of immortalized chimpanzee hepatocyte (CHMP) cells with predominant morphologies characterized by (1A) compact cells exhibiting minimal cytoplasm, (1B) spindle-like cells with cytoplasmic extensions, (1C) flattened cells exhibiting a granular and enlarged cytoplasmic area, and (1D) cuboidal cells exhibiting a morphology resembling normal primary hepatocytes in culture;

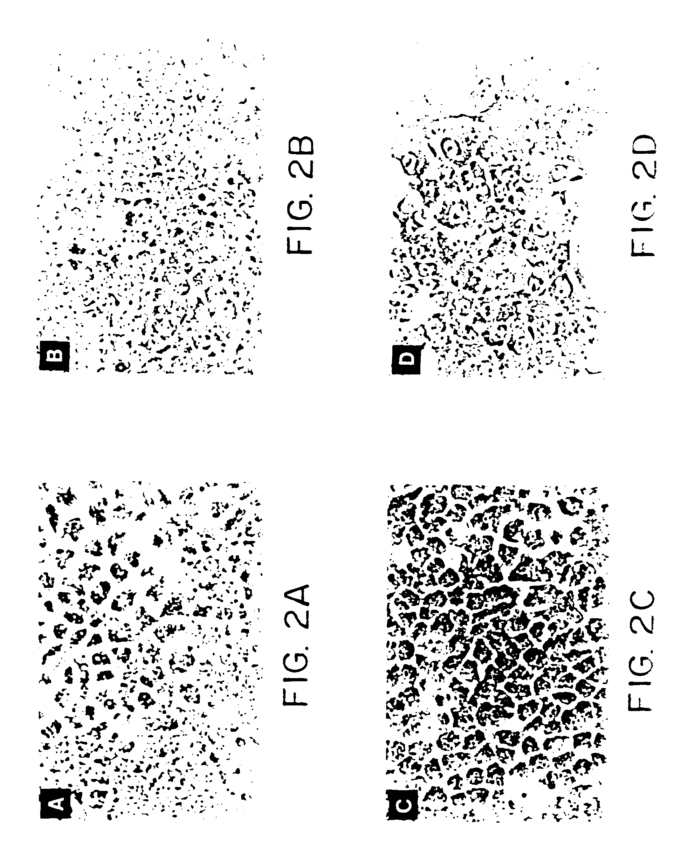

Figures 2A-2D are phase contrast photomicrographs of immortalized baboon hepatocyte cells immortalized with (2A) U19 retrovirus expressing SV40 large T antigen, (2B) a plasmid encoding both SV40 large and small T antigens, (1C) plasmids containing both the myc and ras oncogenes, and (1D) plasmids containing both the E1A and myc oncogenes;

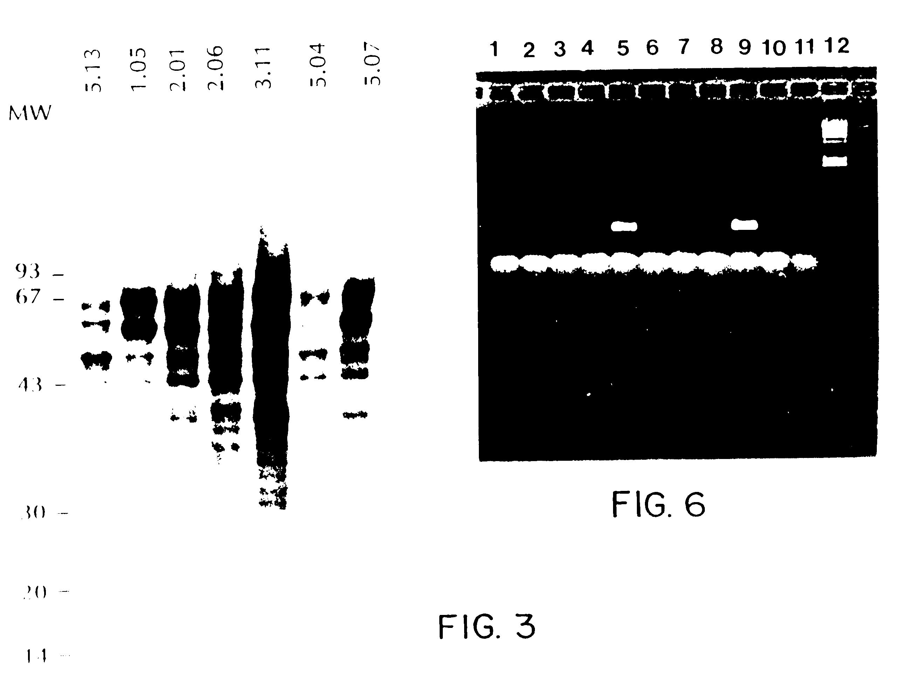

Figure 3 shows electrophoretic patterns of total proteins secreted by the CHMP cell lines indicated at the top of the figure, where the numbers at the left in the figure indicate molecular weights (in kilodaltons) of known marker proteins;

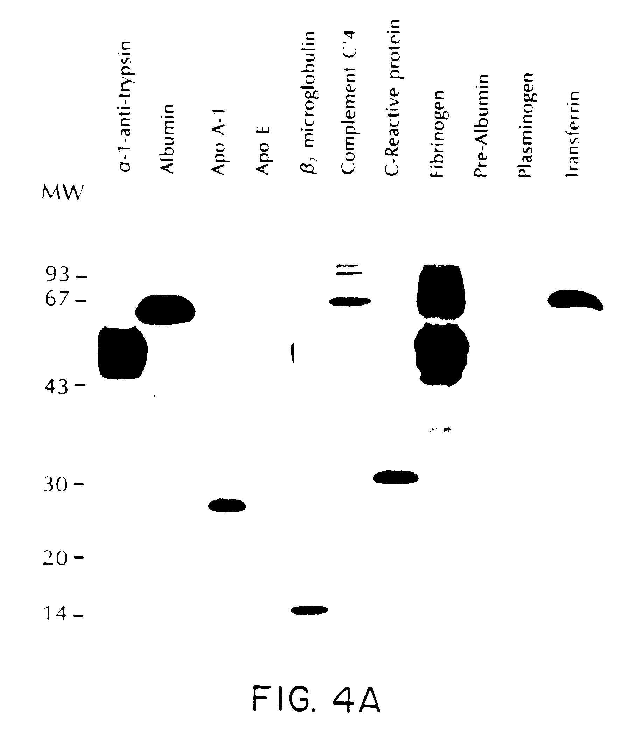

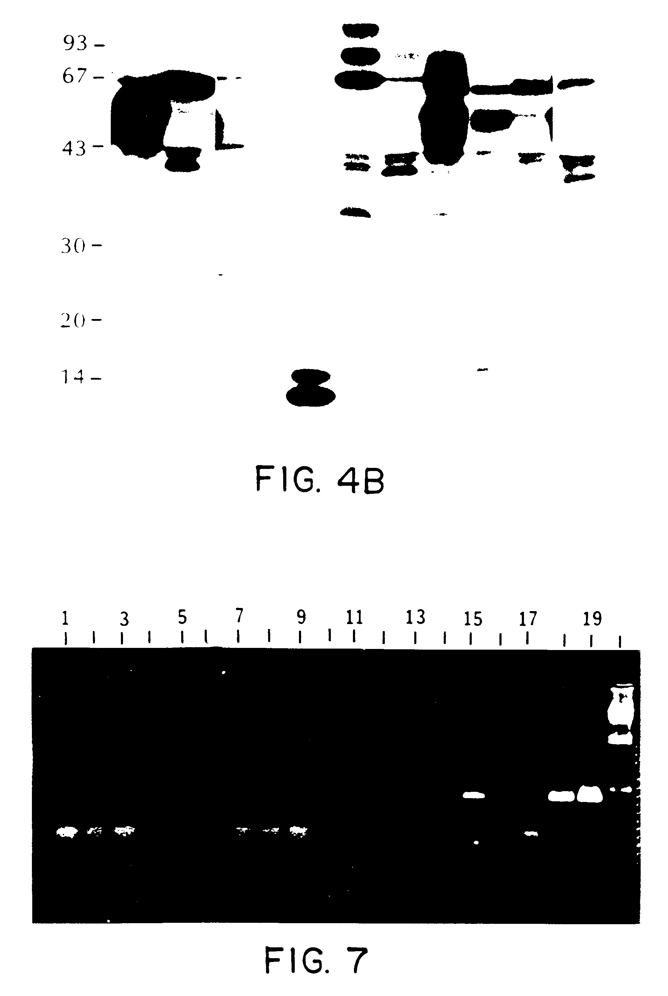

Figures 4A and 4B are gel electrophoretic patterns of proteins secreted by normal chimpanzee hepatocytes (4A) and CHMP 1.20 cells (4B), and prepared by immunoprecipitation of total secreted proteins with antibodies specific against the proteins indicated at the top in the figures, followed by electrophoresis of the precipitated proteins;

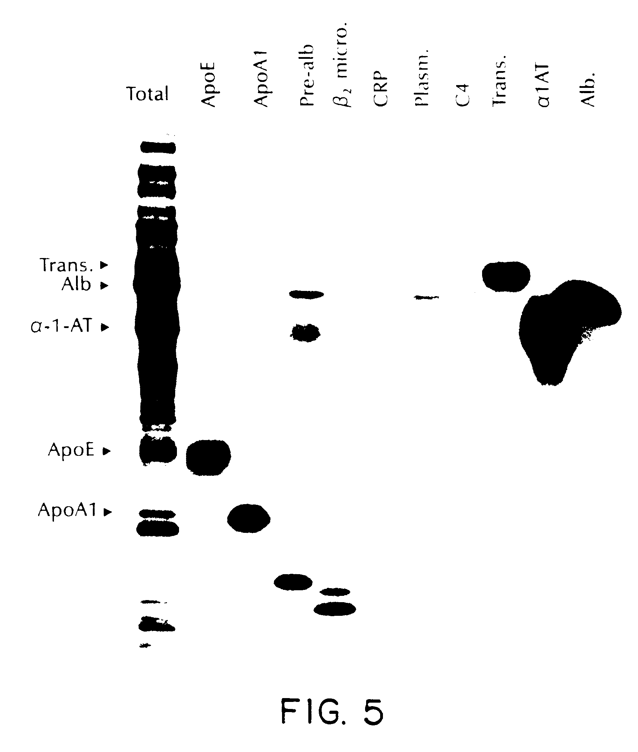

Figure 5 shows gel electrophoresis patterns of proteins secreted by immortalized baboon hepatocytes, and prepared by immunoprecipitation of total secreted proteins with antibodies specific against the proteins indicated at the top in the figure, followed by electrophoresis of the precipitated proteins;

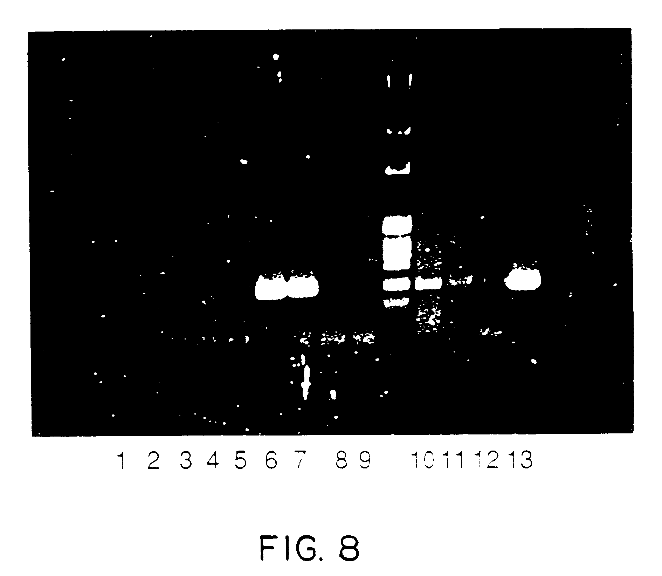

Figure 6 shows electrophoretic patterns of PCR (polymerase chain reaction) products of cellular RNA from various immortalized chimpanzee hepatocyte cell lines (CU cell lines) derived from an HCV-infected chimpanzee (lanes 1-8), and from chimpanzee liver RNA during the acute phase of HCV infection (lane 9);

3

Figure 7 shows electrophoretic patterns of PCR products of RNA from various CHMP cell lines infected in vitro with HCV (lanes 1-12 and 14), from the inoculum used to infect the cells (lane 13), from chimpanzee liver RNA during the acute phase of HCV infection (lanes 15 and 18, and from an HCV cloned fragment; and

Figure 8 shows electrophoretic patterns of PCR products of RNA from various HAV-infected immortalized cells derived from marmoset hepatocytes (lanes 6 and 7), from control HAV inoculum (lanes 10 and 11), and from PCR positive controls (lanes 12 and 13).

Detailed Description of the Invention

I. Immortalization Procedures

This section describes methods for producing an immortalized primate, hepatocyte cell line whose cells have the following characteristics:

(a) an oncogene integrated in the cellular genome;

(b) secretion in culture of several (at least three) liver-specific hepatocyte secretory proteins, such as albumin, $\alpha$-1-antitrypsin, complement C'4, fibrinogen,apolipoproteins A-1 and E, transferrin, and plasminogen; and

(c) ability to support replication of human hepatotropic viruses, such as hepatitis A virus (HAV), hepatitis B virus (HVB), hepatitis E virus (HEV), and hepatitis C virus (HCV).

A. Cultured primary hepatocytes

The immortalized cell line is derived from, i.e., obtained from primary primate hepatocytes which are cultured under conditions which maintain liver-specific functions, particularly the ability to produce and secrete liver-specific proteins, for periods of up to 100 days or more in culture. Methods for preparing primary primate hepatocytes for culture, and culture medium conditions effective to preserve liver-specific functions for extended periods in culture have been described by the inventors (Lanford, 1989). Briefly, liver tissue obtained by liver biopsy from a human, chimpanzee, baboon, marmoset or other primate, is perfused and hepatocytes are dislodged by treatment with collagenase. The cells are washed several times, then plated on culture plates at a density of about $5 \times 105$ to $5 \times 10^6$ cells per 60 mm plate.

The hepatocytes are maintained in serum-free medium (SFM) which has been specifically designed to allow the cells to grow in culture in a liver-differentiated form, as evidenced by the continued production and secretion in culture of liver-specific proteins. One preferred culture medium is composed of Williams' medium E (WME) supplemented with 10 mM HEPES, pH 7.4, 50 ug gentamycin, and the following supplements: EGF (epidermal growth factor), insulin, glucagon, BSA (bovine serum albumin), soybean lipids, linoleic acid, hydrocortisone, selenium, cholera toxin, LGF (liver growth factor, a glysyl-histidyl-lysine tripeptide), ECGS (endothelial cell growth supplement), transferrin, ethanolamine, prolactin, somatotropin, and TRF (thyrotropin-releasing factor), in the proportions given in Example 1. The sources of these materials are given elsewhere (Lanford).

The cells are maintained in the SFM under standard cell culture conditions. The medium is changed, e.g., 24 hours after isolation and every 48 hours thereafter, during the culture period. For some primate hepatocytes, such as derived from chimpanzee liver, the cells appear to undergo 2-4 rounds of replication in the first several days of culture, e.g., within 7-10 days, and thereafter continue to function as liver-specific cells in culture, but without appreciable signs of cell replication. In other primate hepatocytes, such as derived from baboon and marmoset liver, the primary cells also undergo 2-4 rounds of replication in culture, but thereafter, continued cell replication is observed from foci in the original culture plate, and these foci undergo replication until they grow to a monolayer on the plate. These cells in the monolayer are still differentiated hepatocytes, as evidenced by continued production and secretion of liver-specific proteins. This distinction between different types of cultured primary primate hepatocytes is mentioned because the strategy for selecting immortalized cells may vary according to the replication behavior of the cells during the immortalization step.

A variety of methods are available to confirm that the cultured primary cells are producing and synthesizing liver-specific secretory proteins. Two of these methods, involving analysis of total culture medium by sodium dodecyl sulfate polyacrylamide gel electrophoresis (SDS-PAGE) and immunoprecipitation with antibodies against liver-specific proteins, are described in Example 3, with reference to detection of liver-specific products produced by the immortalized liver cells of the invention. Typically, the primary cells will produce serum albumin, $\alpha$-1-antitrypsin, complement C'4 apolipoprotein E and A-1, fibrinogen, plasminogen, transferrin, in combinations including at least three of these proteins.

B. Cell Immortalization

The primary cells from above are immortalized by integrating into the cell genome, an oncogene effective to inhibit the normal cell-replication control mechanism(s) of the primary cells in culture. The sources of a variety of oncogenes, and their mechanism of action in releasing normal cell replication constraints have been reviewed (Bishop). Typically, the oncogenes are isolated from viruses which have picked up an oncogene from a mammalian host genome, and where the oncogene exists in a mutated from which produces an activated, i.e., unregulated gene product effective to overcome a cell's normal growth-inhibition mechanism(s). Table 1 below lists several of the oncogenes which have been described to date in the literature.

Table 1

| Abbreviation | Virus |
|---|---|
| arc | Rous Sarcoma Virus (Chicken) |
| yes | Y73 Sarcoma Virus (Chicken) |
| fps | Fujinami (St. Feline) Sarcoma Virus |
| abl | Abelson Marine Leukemia Virus (Mouse) |
| ros | Rochester-2 Sarcoma Virus (Chicken) |
| fgr | Gardner-Rasheed Feline Sarcoma Virus (Cat) |
| erba | Avian Erythroblastosis Virus (Chicken) |
| fms | McDonough Feline Sarcoma Virus (Cat) |
| mos | Moloney Murine Sarcoma Virus (Mouse) |
| raf | 3611 Murine Sarcoma[+] Virus (Mouse) |
| Ha-ras-1 | Harvey Murine Sarcoma Virus (Rat) (Balb/c mouse; 2 loci) |
| Ki-ras 2 | Kirsten Murine Sarcoma Virus (Rat) |
| Ki-ras 1 | Kirsten Murine Sarcoma Virus (Rat) |
| myc | Avian MC29 Myelocytomatosis Virus (Chicken) |
| myt | Avian Myelo Blastomas (Chicken) |

| Abbreviation | Virus |
|---|---|
| fos | FBJ Osteosarcoma Virus (Mouse) |
| ski | Avian SKV T10 Virus (Chicken) |
| rel | Reticuleondotheliosis Virus (Turkey) |
| sis | Simian Sarcoma Virus (Woolly Monkey) |
| N-myc | Neuroblastomas (Human) |
| N-ras | Neuroblastoma, Leukemia Sarcoma Virus (Human) |
| Blym | Bursal Lymphomas (Chicken) |
| man | Mammary Carcinoma (Human) |
| neu | Neuro, Blioblastoma (Rat) |
| ertA1 | Chicken AEV (Chicken) |
| ra-ras | Rasheed Sarcoma Virus (Rat) |
| mnt-myc | Carcinoma Virus MH2 (Chicken) |
| myc | Myelocytomatosis CK10 (Chicken) |
| myb-ets | Avian myeloblastosis/ erythroblastosis Virus E26 (Chicken) |

The oncogenes can be placed recombinantly in plasmids which are capable of infecting a suitable host cell. Alternatively, the oncogenes can be placed in a suitable virus vector, such as a retrovirus (Miller) vector which is capable of infecting the host cell, with constitutive production of the oncogene product. A variety of plasmid and virus constructs which carry oncogenes and which are capable of introduction into mammalian cells in culture have been reported, and are available, either from public depositories such as the American Type Culture Collection (Rocklawn, MD), or by using recombinant plasmid techniques to form plasmid constructs described in the literature. In the method detailed in Example 1 for the immortalization of chimpanzee primary hepatocytes, and in Example 6 for immortalization of marmoset hepatocytes, the oncogene for the SV40 large T antigen, which is defective for binding to the SV40 origin of DNA replication, was used for immortalization. The oncogene was introduced by way of a U19 retrovirus, whose genome was designed for constitutive expression of the T antigen. The retrovirus can be grown in and harvested from the culture medium of a suitable host, such as the U19-5 cell line described in Example 1.

In the method detailed in Example 5 for the immortalization of baboon hepatocytes, four different oncogenes or oncogene combinations were tested for ability to immortalize the primary cells. In the first method, the cells were infected by the U19 retrovirus containing the oncogene for SV40 large T antigen, described above. In the second-fourth method, the cells were exposed to various plasmid constructs containing either ras, myc, or E1A oncogenes. The second method involved the pSV3neo plasmid containing both large and small SV40 T antigen oncogenes, and a neomycin resistance gene. The third method involved a combination of plasmid pSVc-myc-1, which contains the myc oncogene, and plasmid pUJ EJ 6.6, which contains the ras oncogene. The fourth method involved a combination of the plasmid p1Aneo, which contains the E1A oncogene, and the above plasmid pSVc-myc-1. The last-mentioned method produced cells with highly differentiated morphology, as noted in Example 5, while the first three methods produced cells which appear more transformed and less differentiated.

The oncogene may be introduced into the primary cells using a variety of vectors. Where the oncogene is contained in an infective viral vector, such as the U19 retrovirus noted above, the oncogene is introduced by viral infection, typically by exposing the cells to the virus for a period of several hours, then washing the cells to remove free virus. Where the oncogene is carried on a plasmid, standard methods for introducing plasmids into mammalian cells may be used. These include electroporation, and plasmid uptake in the presence of CaCl2 or lipofection (Felgner). The latter method is preferred, since it allows introduction of plasmids with reasonable efficiency and little cell disruption. Details of retrovirus infection for immortalization of chimpanzee and marmoset hepatocytes are given in Examples 2 and 6, respectively. Immortalization of baboon hepatocytes with plasmids in the presence of lipofection are given in Example 5.

To introduce the oncogene, the hepatocytes are plated at a subconfluent level which allows several rounds of replication after the oncogene vector is introduced. The cells are exposed to the oncogene vector for a period of typically several hours, after which the cells are washed to remove free vector, and then cultured in SFM. As noted above, primate cells will undergo 2-4 rounds of replication in the first 7-10 days under the culture conditions described above. During this period, the culture medium is changed every 1-3 days.

At the end of the period of initial replication, e.g., after 7-10 days in culture, the culture will consist of both immortalized and non-immortalized hepatocytes. At this point, it may be necessary to expose the cells to a selection pressure which allows selective proliferation of the immortalized cells. To this end, the oncogene vector is provided with a selective marker, such as an antibiotic resistance gene, which allows growth selection in the presence of the antibiotic. In the case of chimpanzee hepatocytes, which tend to cease replication at the end of initial 2-4 rounds of replication, it may not be necessary to subject the cells to antibiotic selection, since the immortalized cells will gradually take over the remaining, non-replicating primary cells. Here the cells may be allowed to expand in culture for a period of 3-5 weeks, until colony outgrowths, represented by immortalized cell colonies, are observed. Alternatively, an antibiotic may be added after the initial phase of replication, to insure complete removal of non-immortalized cells from the culture.

In the case of primate hepatocytes, such as baboon or marmoset hepatocytes, which show continued growth of primary cells from foci, after the initial round of replication, the cells are first grown in the absence of antibiotic (or at a low level of antibiotic) for 7-10 days, or until the initial 2-4 rounds of replication occur. At this point, the cells are then exposed to the antibiotic, at a concentration sufficient to selectively kill the non-immortalized primary cells (which do contain the gene for antibiotic resistance). The cells are then cultured further in the presence of the antibiotic until colony outgrowths are observed. The cells are not exposed to antibiotic during the initial rounds of replication to prevent premature killing of cells before expression of the antibiotic resistance gene in the oncogene vector can occur, and to reduce toxicity to the cells in culture by cellular products released from dying cells.

After colony outgrowths of immortalized cells are obtained, the cells are again disrupted, e.g., by collagenase/dispase treatment. The cells are then resuspended in a suitable attachment medium, such as WME (Williams Medium E) supplemented with 5% fetal bovine serum (FBS), and replated at low density. After 3 hours, the cultures are changed to SFM to allow single-colony outgrowths to be isolated. The resultant immortalized colonies are then characterized, to identify cell lines which (a) retain their liver-cell differentiation, as evidenced by production of liver-specific proteins, and (b) are infectable with hepatotropic viruses. These characterization methods are discussed in Section II.

## II. Characterization of the Immortalized Cells

### A. Morphology

The immortalized cells may have one of several distinguishable morphologies which depend on the primate species from which the hepatocytes are derived, and the oncogene(s) used in the immortalization method.

Figures 1A-1D show four of the distinguishable morphologies which have been observed in immortalized chimpanzee hepatocytes, produced by U19 retrovirus immortalization. The Figure 1A cells are compact cells which exhibit minimal cytoplasm. Cells line having this general morphology are represented by the selected cell lines designated CHMP 5.13, CHMP 1.05, CHMP 2.01, CHMP 2.06, and CHMP 3.12. The Figure 1B cells are spindle-like cells with cytoplasmic extensions. Cells having this morphology are represented by the cell line designated CHMP 5.01.

The Figure 1C cells are flattened cells exhibiting a granular and enlarged cytoplasmic area. Representative cell lines include the one designated CHMP 5.03. The fourth cell morphology type has cuboidal cells which resemble normal primary cultured hepatocytes morphologically, as represented by cell line CHMP 5.04.

The variation in cell morphology which can be produced by different oncogenes is illustrated by the four morphology types seen in Figures 2A-2D, which show photomicrographs of baboon hepatocytes immortalized with a retrovirus (2A), or one or more oncogene-containing plasmids. Specifically, the hepatocyte cells immortalized with (2A) U19 retrovirus expressing SV40 large T antigen, (2B) a plasmid encoding both SV40 large and small T antigens, (1C) plasmids containing both the myc and ras oncogenes, and (1D) plasmids containing both the E1A and myc oncogenes. As seen, the ^T cell line (Figure 2D) has a highly differentiated morphology, while the other cell lines appear more transformed and less differentiated.

B. Secretory Proteins

According to an important aspect of the invention, the immortalized primate hepatocytes cells retain hepatocyte differentiation, as evidenced by the ability of the cells to produce several, i.e., at least three, liver-specific secretory proteins, such as albumin, $\alpha$-1-antitrypsin, complement C'4, fibrinogen, apolipoproteins A-1 and E, transferrin, and plasminogen, but also including minor liver-specific proteins, such as C-reactive protein, apolipoproteins a-II, apolipoproteins C2 and C3. Preferably, the cells produce the secretory proteins albumin, $\alpha$-1 antitrypsin, one or both apolipoproteins A-1 and E, complement C'4, and one or both clotting factors plasminogen and fibrinogen.

Preferably, the secretory proteins are made in approximate relative proportion to their concentrations in the serum. That is, the major liver secretory proteins in the plasma, such as albumin, $\alpha$-1 antitrypsin, and transferrin, are the major secretory proteins produced by the immortalized cells, and the minor proteins in the plasma are the minor ones produced in the cell culture.

The presence of liver-specific secretory proteins in the cell culture medium can be confirmed by a variety of methods. In one, described in Example 3, hepatocyte cultures representing compact cell morphology (CHMP 5.13, CHMP 1.05, CHMP 2.01, CHMP 2.06, and CHMP 3.11), flat cell morphology (CHMP 5.13), and cuboidal cell morphology (CHMP 5.04) were incubated with [35] methionine, and radiolabeled proteins from culture medium were fractionated by SDS-PAGE. Figure 3 shows autoradiographs of culture-medium proteins from the several CHMP cell lines. The major proteins detected by this analysis had molecular sizes corresponding to albumin (67kd) and alpha-l-antitrypsin (54kd). The size markers (molecular weight given in kilodaltons) were phosphorylase B (93kd), serum albumin (67kd), ovalbumin (43kd), carbonic anhydrase (30kd), trypsin inhibitor (20kd), and lysozyme (l4kd). Details of the method are given in Example 3.

Secretory proteins in the culture medium can be positively identified by immunoprecipitation methods, such as detailed in Example 3. Here culture medium containing radiolabeled proteins are reacted with immobilized antibodies specific against one of a number of different primate, e.g., human, serum proteins. The immunospecifically bound proteins are then released from the antibodies and fractionated by SDS-PAGE, as detailed in Example 3. The identity of the immunoprecipitated proteins is indicated at the top in Figures 4A and 4B. The profile of secretory proteins obtained with primary hepatocytes (Figure 4A) was very similar to that observed for the CHMP l.20 cell line (Figure 4B), demonstrating the retention of liver-specific functions, i.e., a highly differentiated state, after immortalization.

A similar immunoprecipitation method was used to determine the identity of secretory proteins expressed in several CHMP cell lines. The results are summarized in Table 2. The "+" symbol in the table means normal expression, i.e., expression comparable to that observed in primary cultures; the "*" symbol, low expression,; the "-" symbol, no expression; and "NT", not tested.

Several of the lines, as exemplified by CHMP 1.20, expressed a majority of the plasma proteins investigated. The level of expression of the apolipoproteins A-1 and E in many of the cell lines was similar to that observed in primary cultures, although $\beta_2$-microglobulin and prealbumin expression levels were elevated.

9

Complement C'4 was detected in all the cell lines; however, one or two of the associated chains (alpha or gamma) were not detected by precipitation with the respective antibody. C-reactive protein was not detected in any cell line. CHMP 5.01 was deficient in albumin expression although it still expressed several other plasma proteins including apo E. The one cell line is clearly deficient in differentiated liver cell functions is CHMP 2.03, which produces only $\alpha$-1 antitrypsin among liver-specific proteins.

No attempt was made to investigate the possible intracellular accumulation of plasma proteins which may have occurred due to the transformed state of the culture in question. Unlike the established human hepatocellular carcinoma cell lines, no $\alpha$-fetoprotein has been detected in any primate hepatocyte cell line examined to date.

Similar protein-identification studies were carried out with immortalized baboon cells, as described in Example 6. Figure 5 shows in lane A, total secreted proteins present in the culture medium, after fractionated by SDS-PAGE. The identification of several liver secretory proteins, based on migration distance on the gel, is shown at the left of lane 1. Immunoprecipitation of the radiolabeled culture proteins, and subsequent fractionation by SDS-PAGE, indicates the relative amounts of various proteins, including apolipoproteins E and A-1, pre-albumin, plasminogen, complement C'4, transferrin, $\alpha$-1 antitrypsin, and albumin, as seen.

C. Oncogene Expression

The immortalized cells can be assayed for expression of the oncogene, by similar immunoprecipitation methods for detecting oncogene products in the culture medium. For example, the large T antigen product of the U19 oncogene used to immortalize chimpanzee hepatocytes was identified by immunoprecipitation of the T antigen in each of the immortalized CHMP cells reported in Table 2, as indicated at the right in the table.

Table 2

| | Primaries | 1.20 | 1.33 | 2.02 | 2.03 | 2.05 | 3.01 | 4.03 | 4.07 | 5.01 |
|---|---|---|---|---|---|---|---|---|---|---|
| Albmn | + | + | + | + | – | + | + | + | + | – |
| α1α Tryp | + | + | + | + | + | + | + | + | + | + |
| apo A-1 | + | + | + | * | – | * | – | + | + | – |
| apo E | + | + | + | + | – | * | * | + | + | + |
| $\beta_2$-micro | + | + | + | + | + | + | + | + | + | + |
| C'4 | + | + | + | + | * | + | + | + | + | + |
| CRP | + | – | – | – | – | – | – | – | – | – |
| Fibrngn | + | + | + | + | – | + | – | + | + | – |
| Plsmgn | + | – | – | * | – | – | – | – | – | * |
| PreAlb | + | + | + | * | – | – | – | + | * | * |
| Trnsfrn | + | + | + | * | – | * | + | + | + | + |
| AFP | – | – | – | – | – | – | – | – | – | – |
| T-ag | N.T. | + | + | + | + | + | + | + | + | + |

Immunoprecipitation of liver specific plasma proteins:
+ – Normal Expression
* = Low Expression
: No Expression
N.T. = Not Tested

Several of the lines, as exemplified by CHMP 1.20, expressed a majority of the plasma proteins investigated. The level of expression of the apolipoproteins A-1 and E in many of the cell lines was similar to that observed in primary cultures, although $\beta_2$-microglobulin and prealbumin expression levels were elevated.

Complement C'4 was detected in all the cell lines; however, one or two of the associated chains (alpha or gamma) were not detected by precipitation with the respective antibody. C-reactive protein was not detected in any cell line. CHMP 5.01 was deficient in albumin expression although it still expressed several other plasma proteins including apo E. The one cell line that is clearly deficient in differentiated liver cell functions is CHMP 2.03, which produces only α-1 antitrypsin among liver-specific proteins.

No attempt was made to investigate the possible intracellular accumulation of plasma proteins which may have occurred due to the transformed state of the culture in question. Unlike the established human hepatocellular carcinoma cell lines, no α-fetoprotein has been detected in any primate hepatocyte cell line examined to date.

Alternatively, the presence of the oncogene in the immortalized cells can be determined directly, by PCR amplification of the oncogene sequences in a cell genomic digest. This approach was used to confirm the presence of the oncogene in the cell genome in a number of immortalized cell lines.

D.. Infectivity by Hepatotropic Viruses

Another important property of the immortalized primate hepatocytes of the present invention is the ability to support replication of human hepatotropic viruses. By this is meant that the cell line can be infected with an active hepatitis virus, and the virus can replicate in the cultured cells, with the production of mature virus particles, preferably in the culture medium.

One hepatitis virus of particular interest, because of its importance as a blood-borne pathogen, is HCV. The ability of CHMP cells to support HCV infection is demonstrated from the studies reported in Example 4. Here CHMP cells (prepared by immortalization of non-infected chimpanzee hepatocytes) and CU cells (similarly prepared from HCV-infected chimpanzee hepatocytes) were both inoculated with chimpanzee plasma known to contain HCV, as detailed in Example 4. On the 11th day after infection, the cultures were harvested for analysis.

To confirm the presence of HCV in the infected cells, total RNA from the cells was amplified by PCR methods, using probes with known HCV sequences. Details are given in Example 4. Figure 6 shows a gel analysis of the PCR products from HCV-infected CU cell lines. Lanes l-8 are CUl, CU3, CU4, CU5, CU6, CU8, CU9 and CUl2, respectively. Lane 9 is a positive control of chimpanzee xl98 liver RNA during the acute phase of HCV infection and was processed identically as the CU RNA samples. Lanes lO and ll are the cDNA and PCR negative controls to demonstrate the lack of contamination during the PCR assay. Lane l2 is lambda DNA cleaved with HindIII as size markers. Lane 5 (CU6) and 9 (PCR positive control) show a positive reaction. All lanes have a lower band that represents the primers used in the PCR reaction.

Figure 7 shows a gel analysis of PCR products from HCV-infected CHMP cell lines. Lanes l-l2 represent CHMP l.21, l.22, l.23, l.24, l.25,l.26, l.27, l.28, l.29, l.30, l.3l and l.32, respectively. Lane 13 is the PCR analysis of the inoculum used to infect both the CU and CHMP cell lines. Lane l4 is CHMP 2.02. Lane l5, l8 and l9 are PCR positive controls. Lane l5 and l8 are xl98 liver RNA as described for Figure 6. Lanes l6 and l7 are cDNA and PCR negative controls, respectively. Lane l9 is a PCR positive control consisting of a gel purified band from a cloned fragment of HCV homologous to the PCR primers used in this assay. Lane 2O is HindIII digested lambda DNA as size markers.

Positive reactions were obtained with CHMP l.27 and 2.02, the inoculum used to infect the cell lines, and each of the positive controls. The negative controlled were negative indicating that no contamination occurred during the PCR reaction.

Thus, of the 20 CU and CHMP cell lines tested, three have been shown to be permissive for infection with and replication of HCV. The cell lines are CHMP l.27, CHMP 2.O2 and CU6. These cell lines are selected as meeting both criteria of the cells of the present invention: (a) secretion of liver-specific proteins, and (b) ability to support hepatitis virus infection.

These results demonstrate that the immortalized cell lines of the invention are infectable with HCV, and support replication of HCV, for use in the production of HCV for use in diagnostics and vaccines, and the use of the infected cell lines for testing antiviral compounds for the inhibition of HCV replication.

Infection of immortalized primate hepatocytes by HAV is demonstrated in the study reported in Example 7, which shows HAV infection and growth in immortalized marmoset hepatocytes. After inoculating the cells with a HAV inoculum, total cellular RNA was isolated by the method as described above and the samples were analyzed by PCR, using probes with HAV-specific sequences. Figure 8 shows the gel analysis of PCR products from HCV-infected CJSl cell line. Lane l is PCR negative control; lanes 2-5, uninfected CJSl cells; lanes 6 and 7, HAV-infected CJS1 cell lines with 5% of the nucleic acid being examined by PCR; lanes 8 and 9, the HAV negative inoculum used for infection of CJSI in lanes 4 and 5; lanes lO and ll, PCR analysis of the inoculum for lanes 6 and 7; lane l2, weak PCR positive control; and lane l3, strong PCR positive control. The positive controls were positive, the negative controls were negative, the uninoculated cells were negative, the inoculated cells were positive, and the inoculum was positive. As seen, one of the cell lines, CJS1, was infectable by HAV.

It will be appreciated that the immortalized hepatocytes of the invention can be infected by other hepatotropic viruses, such as HBV, and enterically transmitted Non-A, Non-B hepatitis virus, for viral replication in the cells. The methods described above indicate that immortalized primate hepatocytes capable of supporting hepatitis viral replication in culture occur at a frequency which allows their selection from a relatively small number of possible cell lines.

12

III. Utility

A. Virus Particles Production

As demonstrated above, the immortalized cells of the present invention are infectable with and support replication of human hepatotropic viruses, such as hepatitis viruses. The cells can therefore be used to produce such viruses in culture. As examples, immortalized chimpanzee or human hepatocytes can be used to produce HCV virus particles. A variety of primate hepatocytes, including marmoset hepatocytes, can be used to produce HAV particles.

In a typical method, an immortalized hepatocyte cell line capable of supporting replication of the hepatotropic virus is selected, for example, by detection of viral RNA in the infected cells, as described above. The cell line is infected with the virus, typically by inoculating the cells with plasma from humans infected with the virus of interest, also as detailed above. Virus infection of the cells can be confirmed by PCR methods for detecting virus-specific RNA, described in Example 3, or by showing that the culture medium is itself infective for primary or immortalized hepatocytes.

Virus particles in the culture medium can be isolated by a variety of available methods. In one approach, detailed in the companion PCT application for "Purified Non-A, Non-B Hepatitis Virus", culture medium harvested from the infected cells was first clarified by low-speed centrifugation (12,000 x g for 30 minutes), and the clarified material was layered over a discontinuous 20% sucrose and 68% sucrose layers, and centrifuged at high speed (131,000 x g) for 3.8 hours. The material at the 20%/60% interface is collected, diluted with buffer, then centrifuges a second time over a 68% sucrose cushion at 154,000 x g for 16 hours. The material at the interface contains the desired HCV particles, as demonstrated by PCR methods and electron microscopic analysis. Similar centrifugation methods may be applied to obtain other hepatitis virus particles in purified or partially purified form.

Alternatively, the virus particles may be isolated from the culture supernatant by affinity chromatography methods, using immobilized anti-virus antibodies, such as monoclonal antibodies prepared against the virus particles, to capture the particles on a solid support.

The isolated virus particles can be used, in attenuated or inactivated form, in a vaccine composition, or as a diagnostic reagent. The virus particles may alternatively be subfractionated into component mature viral proteins, for use in a vaccine composition or as a diagnostic reagent.

B. Drug Screening

Immortalized cells which are infected with a selected hepatotropic virus, in accordance with the invention, may be employed in a drug screening method, for identifying drug compounds effective to inhibit the growth of the virus in hepatocytes. In this method, a culture of infected cells are exposed to the test compound, typically over a selected drug concentration range, for a given inhibition period, typically 6-48 hours. Thereafter, the cells, or virus particle obtained from the cells, are examined for level of virus.

One general method for quantitating virus levels in infected cells is described in Example 3, for detection of HCV infection in immortalized chimpanzee hepatocytes. Here total RNA is isolated from infected cells, for amplification by standard polymerase chain reaction (PCR) methods, employing known, virus-specific sequences. The level of RNA can be quantitated, for example, by dot-blot hybridization (Sambrook).

Virus levels can be quantitated by measuring the levels of virus-specific cell-surface antigens. U.S. Patent No. 4,777,245 describes an IgM monoclonal antibody which is specific against cell surface antigen in HCV-infected liver cells. The level of antibody binding to the cells can be quantitated, for example, by a standard enzyme immunoassay methods employing an enzyme-labeled antibody specific against the first-bound antibody.

From the measured inhibition of virus growth in infected, treated cells, compounds which are likely candidates for in vivo testing can be identified.

C. Secretory Protein Production

One of the important features of the immortalized cell lines of the invention is the ability to synthesize liver-specific secretory proteins, such as albumin, $\alpha$-1-antitrypsin, complement C'4, fibrinogen, apolipoproteins A-1 and E, transferrin, and plasminogen. As noted above, there is some variation in the type and levels of various serum proteins which are synthesized by different immortalized cell lines. This suggests that immortalized cells with highly specific protein-synthesis specificity can be identified, for use

13

in production of selected serum proteins.

The immortalized cells are grown on a suitable growth medium, such as the SFM medium described above, which maintains the differentiated state of the cells. The selected secretory proteins are obtained from culture medium, and isolated from the medium by standard protein fractionation methods.

The following examples illustrate specific methods for producing immortalized primate hepatocytes, and for characterizing the morphology, cell products and viral infectivity of the cells. The examples are intended to illustrate, but not limit, the scope of the invention.

Example 1

Primary Hepatocyte Cells

A. Cultured Primary Chimpanzee Hepatocytes

Liver cells were obtained from a five-year-old male chimpanzee (Pan troglodytes) (Joshua (PTTx266), housed at a primate facility. The animal had not been previously exposed to hepatitis A virus, hepatitis B virus, delta hepatitis virus, non-A, non-B hepatitis virus, or human immunodeficiency virus. Liver wedge biopsy was performed as described by Eichberg. Hepatocytes were isolated by standard perfusion and collagenase treatment of the liver wedge as has been described (Lanford, 1989).

In this and the other examples below, the serum-free media (SFM) formulation utilized a basal medium supplemented with 10 mM HEPES, pH 7.4, 2.75 mg/ml NaHCO$_3$, and 50 $\mu$g/ml gentamicin, together with the supplements as listed below. In the described media of Table 3, Williams Medium E (WME) served as a basal medium. Although WME is presently preferred as the basal medium of the serum-free medium other commercial media formulations can be expected to give satisfactory results. For instance, a mixture of Dulbecco's modified Eagle's medium and Ham's F12 medium (Salas-Prato) or RPMI 1640 (Gibco) (Enat, Sell) should give satisfactory results when supplemented with the supplements listed in Table 3.

Table 3

| Supplement | Medium Concentration |
|---|---|
| EGF | 100 ng/ml |
| Insulin | 10 $\mu$g/ml |
| Glucagon | 4 $\mu$g/ml |
| BSA | 0.5 mg/ml |
| Linoleic Acid | 5 $\mu$g/ml |
| Hydrocortisone | $10^{-6}$ M |
| Selenium | $10^{-8}$ M |
| Cholera Toxin | 2 ng/ml |
| LGF | 20 ng/ml |
| Transferrin | 5 $\mu$g/ml |
| Ethanolamine | $10^{-6}$ M |
| Prolactin | 100 ng/ml |
| Somatotropin | 1 $\mu$g/ml |
| TRF | $10^{-6}$ M |

To prepare the media, the supplements were added in the following quantities in Table 3 to 500 ml of WME in a sterile plastic bottle:

5 ml 50 mg/ml BSA (bovine serum albumin), 500 $\mu$g/ml Linoleic Acid

0.5 ml 5 mg/ml Insulin

0.5 ml 5 mg/ml Insulin,

5 mg/ml Transferrin, and 5 $\mu$g/ml Selenium (ITS)

50 $\mu$l $10^{-2}$ M Hydrocortisone

5 $\mu$l 200 $\mu$g/ml Cholera toxin

0.5 ml 100 $\mu$g/ml EGF (epidermal growth factor)

50 $\mu$l $10^{-2}$ M Ethanolamine

0.5 ml 1 mg/ml Somatotropin

50 $\mu$l 1 mg/ml Prolactin

14

0.5 ml $10^{-3}$ M Thyrotropin Releasing Hormone
50 $\mu$l 200 $\mu$g/ml LGF (liver growth factor, i.e., glycylhistidyl-lysine)
1 ml 2.0 mg/ml Glucagon

WME was purchased with L-glutamine and without $NaHCO_3$ from Hazelton Research Products, Inc. (Denver, Pennsylvania). Supplements, including growth factors and hormones were obtained from Sigma (St. Louis, MO) or Collaborative Research (Bedford, MA).

Dissociation of primary cells and subsequent passages utilized a collagenase/dispase (Boehringer Mannheim) solution at a concentration of 100 $\mu$g/ml in phosphate-buffered saline (PBS, pH 7.2). Following dissociation, a five-fold excess of 5% fetal bovine serum in Williams medium E (5% FBS/WME) was added to the solution. Cells were pelleted at 50 x g for six minutes, resuspended in a minimal volume of 5% FBS/WME and allowed to attach during a 2-3 hour period at 37°C, 10% $CO_2$. Cells were maintained with SFM and changed at two-day intervals.

Under the culture conditions described, the primary cells maintain the characteristics of highly differentiated hepatocytes, as evidenced by cell morphology, and the ability to produce and secrete several liver-specific secretory proteins, including albumin, $\alpha$-1-antitrypsin, complement C'4, fibrinogen, apolipoproteins A-1 and E, transferrin, and plasminogen. The characteristics and stability of the primary cultured cells have been reported by the inventors (Lanford, 1989).

Example 2

Immortalizing Chimpanzee Primary Hepatocytes

The U19-5 cell line which constitutively produces the U19 amphoteric retrovirus was a gift from Drs. P.S. Jat and P.A. Sharp, M.I.T. (Cambridge, MA). The retrovirus construct has been described in detail (Jat). The construct produces a large T antigen protein defective for binding to the SV40 origin of DNA replication.

The U19-5 cell line was grown in DMEM medium (Dulbecco's modified minimal medium, available from Gibco, Grand Island, NY) with 10% FBS (fetal bovine serum) under standard culture conditions (Jat, 1986). Culture medium was collected at 24-hour intervals and passed through a 0.45 $\mu$m filter (Amicon, Beverly, MA) prior to use for infection of primary hepatocyte cultures.

Subconfluent cultures of primary hepatocytes (Example 1) were infected one day post-plating by the addition of 1 ml of U19-5 culture medium to the cells in the presence of Polybrene™ (8 $\mu$g/ml). The plating density was such as to allow the cells several rounds of cell division to occur after introduction of the oncogene. After incubation overnight, cells were washed three times with WME and maintained in SFM until colony outgrowths were observed, typically about 1 month after infection.

The cells were selected for G418 resistance by addition to the culture medium of G418 (Geneticin, GIBCO) (400 $\mu$g/ml). The cells were then treated by a collagenase/dispase (Boehringer Mannheim) solution at a concentration of 100 $\mu$g/ml in phosphate-buffered saline (PBS, pH7.2) for 10 minutes at 37 oFC. Following dissociation, a five-fold excess of 5% fetal bovine serum in Williams medium E (5% FBS/WME) was added to the solution. Cells were pelleted at 50 x g for six minutes, resuspended in a minimal volume of 5% FBS/WME and allowed to attach during a 2-3 hour period at 37°C under 10% $CO_2$. The cells were plated at a low cell density so that single colony outgrowths could be isolated and subcloned. From over 100 colonies, over 70 were picked based upon differences in morphological appearance. The cells are designated CHMP cells, and are assigned cell line numbers, such as CHMP 1.21, CHMP 1.22, etc.

Example 3

CHMP Cell Properties

A. Morphological Characteristics

Immortalized cell lines with several different were morphologies were observed. Four dominant morphologies are seen in the photomicrographs (225 magnification) in Figures 1A-1D, as described above.

B. Secretory Proteins

Hepatocyte cultures representing compact cell morphology (CHMP 5.13, CHMP 1.05, CHMP 2.01, CHMP 2.06, and CHMP 3.11), flat cell morphology (CHMP 5.13), and cuboidal cell morphology (CHMP 5.04) were incubated with 100-250 uCi [$^{35}$S]-methionine (.800 Ci/mmol, ICN, Costa Mesa, CA) for 48 hours. A portion of the culture medium from each cell line was fractionated by sodium dodecyl Sulfate-polyacrylamide gel electrophoresis (SDS-PAGE) in 0.1% SDS, 12% acrylamide, according to standard procedures (Laemmli). The gels were developed at -70°C on Kodak XAR-5 film for 2 days, with the results shown in Figure 3. The major proteins detected by this analysis had molecular sizes corresponding to albumin (67kd) and alpha-l-antitrypsin (54kd). The size markers (molecular weight given in kilodaltons) were phosphorylase B (93kd), serum albumin (67kd), ovalbumin (43kd), carbonic anhydrase (30kd), trypsin inhibitor (20kd), and lysozyme (l4kd).

In another study, proteins secreted by normal chimpanzee primary hepatocytes (Example 1) and the CHMP I.20 cell line were compared by immunoprecipitation from labeled culture medium. Antibodies against human serum albumin, $\alpha$-1 antitrypsin, apolipoproteins A-1 and E, $\beta$-2 microglobulin, complement C'4, C-reactive protein, fibrinogen, prealbumin, plasminogen, and transferrin, were obtained from Calbiochem, San Diego, CA).

Immunoprecipitation was carried out according to published procedures (Jacob, 1989). Briefly, the labeled medium was incubated l6 hr at 4°C with antibodies specific to the various plasma proteins bound to protein A agarose beads (BRL, Gaithersburg, MD). The beads were washed three times to remove unbound proteins and the bound proteins were eluted with SDS-gel electrophoresis sample buffer. The samples were analyzed by l2% SDS-PAGE and fluorography, as described above. The identity of the immunoprecipitated proteins are indicated at the top of the figure. The profile of secretory proteins obtained with primary hepatocytes (Figure 4A) was very similar to that observed for the CHMP I.20 cell line (Figure 4B), demonstrating the retention of liver-specific functions, i.e., a highly differentiated state, after immortalization.

In a third study, CHMP cell lines 1.20, 1.33, 2,02, 2.03, 2.05, 3.01. 4.03, 4.07, 5.01 were cultured in the presence of $^{35}$S-methionine, as above, for 48 hours when the cultures were 80-90% confluent. Culture medium from each cell line was immunoprecipitated with the immobilized anti-human serum protein antibodies noted above, plus an antibody specific against $\alpha$-fetoprotein. The immunoprecipitated proteins were separated by SDS-PAGE and the gels developed by autoradiography. Table 2 above summarizes the plasma proteins expressed from the representative CHMP cell lines compared with that observed in normal primary cultures. The "+" symbol in the table means normal expression, i.e., expression comparable to that observed in primary cultures; the "*" symbol, low expression,; the "-" symbol, no expression; and "NT", not tested.

C. Oncogene Expression

Each of CHMP cell lines 1.20, 1.33, 2,02, 2.03, 2.05, 3.01. 4.03, 4.07, 5.01 was also examined for the presence of integrated U19 provirus T antigen/neomycin genes. This was done by digestion of total cellular nucleic acids with the restriction enzyme BamHI, under standard digest conditions (Maniatis). This digestion releases the SV40 T antigen gene which is placed between BamHI sites in the retroviral plasmid construct. The number of proviral inserts can be determined by hybridization to the neomycin gene, since one BamHI site is contributed by flanking cellular sequences. Digested nucleic acids were separated by electrophoresis on 1% native agarose gels. After capillary transfer to nitrocellulose membranes, filters were baked at 80°C for two hours. Hybridization to the neomycin gene was performed at 65°C in a 0.01% Denhardts solution (Denhardt) containing 1% SDS and 1M NaCl. The plasmid construct pSV3neo, which contains the SV40 T antigen and neomycin gene sequences, was nick-translated and used a the hybridization probe. The methods follow conventional techniques (Sambrook, Southern). Hybridization to the nick-translated pSV3neo probe was observed in each of the cell lines.

In addition, each of the cell lines was examined for the presence of T antigen in $^{35}$-methionine labeled cell lysates, using immunoprecipitation with immobilized anti-T antigen antibody. As shown at the right in Table 3, T antigen was present in each of the immortalized cell lines.

Example 4

HCV Infection of CHMP Cells

A. Viral Inoculation

Immortalized chimpanzee hepatocytes derived from HCV-infected primary hepatocytes were prepared substantially as described in Example 2, but using hepatocytes obtained from a liver biopsy of a chimpanzee during acute-phase HCV infection. The cell lines are designated CU cell lines.

Several CHMP and CU cell lines were cultivated on collagen coated 25 cm$^2$ Primaria flasks in SFM under normal conditions (37 °C, 10% CO$_2$ atmosphere). When the cultures reached a level of 90% confluency, they were inoculated with chimpanzee plasma known to contain HCV.

The inoculum was a pool of plasmas obtained from three chimpanzees (x7, x268, and xl74) during the acute phase of a HCV infection and did not contain any other infectious agent. The plasmas were diluted 5-fold in SFM and l ml was added to the cultures. After incubation for 3 hr at 37 °C, another 3 ml of SFM was added to the cultures and the incubation was continued for l6 hr. The cultures were washed three times with WME to remove the inoculum and SFM was added. The medium was changed every other day and on the 11th day after infection the cultures were harvested for analysis.

B. RNA Characterization

The cells were washed three times with phosphate buffered saline (PBS) and the cellular RNA was extracted and purified using a standard GITC extraction procedure (Chomozynski). The cells were lysed with a solution containing 4M guanidine isothiocyanate, O.18% 2- mercaptoethanol, and 0.5% sarcosyl. The cell lysate was extracted several times with acidic phenol-chloroform- isoamyl alcohol, and the RNA was precipitated with isopropanol. The purified RNA was resuspended in water and one tenth of each sample was used for polymerase chain reaction (PCR) amplification to detect the HCV RNA genome.

PCR was conducted using standard methodology (Innis). The first step involved a cDNA reaction in which a DNA copy of the HCV RNA was made using reverse transcriptase and an oligonucleotide primer designated 6A that is complementary to the strain of HCV used in our studies. The four primers used for cDNA and PCR were derived from the putative nonstructural region of HCV designated NS3 and their sequences are given below.

Primers:

5A 5' TCTGTGATAGACTGCAACACG 3'

6A 5' TTTGGTGATTGGGTGCGTCAG 3'

5B 5' GATGCTGTCTCCAGGACTCAA 3'

6B 5' AACAGCGCCCAGTCTGTATAGCAG 3'

The sequence of these primers was derived from the sequence of a cDNA clone of a strain of HCV as previously described (Jacob). A portion of the cDNA reaction mixture (1/4th) was PCR amplified for 35 cycles using the Taq polymerase and the oligonucleotide primers 5A and 6A. A portion of the first round of PCR (1/50th) was used for a second round of PCR using the primers 5B and 6B.

Figure 6 shows a gel analysis of the PCR products from HCV-infected CU cell lines. Figure 7 shows a gel analysis of PCR products from HCV-infected CHMP cell lines. The gel results are discussed above.

Example 5

Immortalized Baboon Hepatocytes

A. Immortalization Method

Plasmid p1Aneo which contains the adenovirus E1A oncogene was obtained from Drs. E. Ruley and K. Maruyana, Cancer Research, Department of Biology, Massachusetts Institute of Technology. The plasmid construction has been described (Van de Woude). Plasmid pSVc myc-1 that contains the myc oncogene was obtained from the American Type Culture Collection (Rocklawn, MD), and is identified by ATCC No. 41029. Plasmid pUJ EJ 6.6 that contains the ras oncogene was also obtained from the American Type Culture Collection, and is identified by ATCC No. 41028.

Primary baboon hepatocytes were prepared from a 2 year old female baboon housed at the Southwest Foundation for Biomedical Research (San Antonio, TX). Hepatocyte cells were obtained from a liver wedge biopsy and cultured to form a stable primary hepatocyte cell line in SFM, as detailed in Example 1. The primary hepatocyte cells retained liver-specific function for several months in culture, as judged by the ability to secrete liver-specific proteins.

U19 retrovirus or plasmid(s) containing selected oncogenes were introduced into the cells by retrovirus infection, in the case of the U19 retrovirus, or by lipofection, in the case of plasmids, according to the manufacturer's protocol (BRL, Gaithersburg, MD). Briefly, lipofection was performed by combining 5 $\mu$g of plasmid DNA for each plasmid, such as 5 $\mu$g of pSVc myc-1 and 5 $\mu$g of pA1neo plasmid, in 1.5 ml of serum-free medium (SFM) with 30 $\mu$g of Lipofectin™ in 1.5 ml of SFM and adding to a 60 mm culture of subconfluent baboon hepatocytes. The mixture was incubated with the cells for 6-16 hours and the medium then removed by washing. The cells were maintained in SFM for 7-10 days and then G418 (100-400 $\mu$g/ml) for 2 weeks in SFM. Single colonies were then treated by a collagenase/dispase (Boehringer Mannheim) solution at a concentration of 100 $\mu$g/ml in PBS, pH 7.2 for 10 minutes at 37°C. Following dissociation, a five-fold excess of 5% fetal bovine serum in Williams medium E (5% FBS/WME) was added to the solution. Cells were pelleted at 50 x g for six minutes, resuspended in a minimal volume of 5% FBS/WME and allowed to attach during a 2-3 hour period at 37°C under 10% $CO_2$. Cell lines established from single colony outgrowths and representing various oncogenes used to immortalize the primary hepatocytes were selected. The cells are designated BH cells, and are assigned cell line numbers, such as BH1A, BH3A, etc.

B. Cell Morphology

Phase contrast photomicrographs were taken of the living cell lines growing on collagen coated plastic surfaces, and representative cell types are shown in Figures 2A-2D. The different morphologies represent those obtained when different oncogenes are used to immortalize baboon hepatocyte. Figure 2A shows cell line BHIA immortalized with the UI9 retrovirus; Figure 2B, cell line BH6A immortalized with a plasmid encoding both SV40 large and small T antigens, pSv3neo; Figure 2C, cell line BH5A immortalized with plasmids encoding both the myc and ras oncogenes; and Figure 2D, cell line BH3A immortalized with plasmids encoding both the myc and E1A oncogenes.

C. Secretory Proteins

Immortalized baboon cell line BH3A was examined for secretory proteins, substantially according to the method described in Example 2. Briefly, a 25 cm$^2$ flask of BH3A cells were labeled for 24 hours with $^{35}$S-methionine and the labeled medium was harvested and clarified. An aliquot of the clarified medium was analyzed directly by SDS-PAGE. For immunoprecipitations, 300 $\mu$l of the labeled medium was incubated for 16 hours with protein A agarose beads containing bound IgG antibodies against specific liver proteins, as described in Example 2. After three washes to remove unbound protein, the proteins were eluted from the beads with SDS gel-electrophoresis buffer, and the eluted material was fractioned by SDS-PAGE and analyzed by autoradiography. Figure 5 shows the pattern of liver secretory proteins produced by the cell line, where the abbreviations at the top of the figure are for apolipoproteins E and A1, prealbumin, $\beta_2$-microglobin, C-reactive protein, complement C'4, transferrin, $\alpha$-1-antitrypsin, and albumin. It is seen from the figure that the BH3A cell line produces all of the liver-specific proteins except C-reactive protein.

Example 6

Immortalized Marmoset Hepatocytes

A liver wedge biopsy was performed on a marmoset. A liver wedge of approximately 5 gm was perfused with collagenase, using standard methods. Viable hepatocytes were plated on collagen-coated Primaria plates (Falcon, Lincoln Park, NJ). The primary hepatocytes were maintained in SFM medium as described in Example 1.

The hepatocytes were immortalized by the U19 retrovirus, using the procedures described in Example 2. Several of the cell lines, including one designated CJS1, were shown to retain their differentiated liver functions, as evidenced by secretion into the cell medium of several liver-specific proteins.

Example 7

HAV Infectivity of Immortalized Marmoset Cells Line

The immortalized marmoset hepatocyte cell line CJS1 was grown on collagen coated Primaria 25cm$^2$ flasks in SFM as described above. When the cultures reached 9O% confluency they were infected with the HMI75 strain of HAV (obtained from Dr. Mary Estes, Baylor College of Medicine, Houston, TX). The virus stock was diluted five-fold in SFM and added to the cultures. The cultures were incubated for 3 hr at 37°C with the inoculum, and then l.5 ml of SFM was added to the cultures and the incubation was continued for l6 hr. The cultures were washed three times with WME to remove residual inoculum and changed to SFM. The medium was changed every other day and the cultures were harvested on day l0 post-infection.

Total cellular RNA was isolated by the method described above and the samples were shipped to Dr. Estes laboratory for PCR analysis. PCR was performed as described above on 5% of the RNA sample, and

EP 0 527 814 B1

the PCR products were analyzed on a l.5% seakem agarose gel. Figure 8 shows the gel analysis of PCR products from HAV-infected CJSI cell line, as discussed above.

Although the invention has been described with respect to particular cell lines and infective agents, it will be apparent that hepatocytes from other primate species, such as from humans, and other hepatotropic viruses, such as hepatitis B can be employed in the invention.

## Claims

1. An immortalized primate hepatocyte cell line whose cells are characterized by:
   (a) an oncogene integrated in the cellular genome;
   (b) secretion in culture of at least three hepatocyte secretory proteins selected from the group consisting of albumin, $\alpha$-1-antitrypsin, complement C'4, fibrinogen, apolipoproteins A-1 and E, transferrin, and plasminogen; and
   (c) ability to support replication by human hepatotropic viruses.

2. The cell line of claim 1, wherein the relative proportion of such secretory proteins produced by the cells is similar to that found in primate serum.

3. The cell line of claim 2, wherein the cells secrete albumin, $\alpha$-1 antitrypsin, apolipoprotein-E, and fibrinogen.

4. The cell line of claim 3, which is derived from primary chimpanzee hepatocytes, and the cells in the cell line are infectable with and support replication of hepatitis C virus.

5. The cell line of claim 1, which is derived from a primary human or chimpanzee hepatocytes, and the cells in the cell line are infectable with and support replication of hepatitis C virus.

6. The cell line of claim 1, which is infectable with and supports replication of hepatitis B or hepatitis A viruses.

7. The cell line of claim 1, wherein the oncogene is selected from the group consisting of SV40 large or small T antigen, adenovirus E1A, myc, ras, or combinations of these.

8. The cell line of claim 1, wherein the cell line is derived from chimpanzee or human primary hepatocytes, and the oncogene is an SV40 large T antigen.

9. The cell line of claim 1, wherein the cell line is derived from baboon primary hepatocytes, and the oncogene is a combination of E1A and myc oncogenes.

10. A method of producing hepatotropic virus particles, comprising
    culturing the immortalized primate hepatocyte cell line of claim 1, after cell infection with the hepatotropic virus, and
    harvesting the virus particles from the culture medium.

11. The method of claim 10, wherein the cell line is derived from human or chimpanzee hepatocytes, and the virus is hepatitis C virus (HCV).

12. The method of claim 11, wherein the cells are infected with HCV by exposing the cells in culture to chimpanzee or human HCV-infected serum.

13. The method of claim 10, wherein the cell line is derived from chimpanzee or human primary hepatocytes, and the oncogene is an SV40 large T antigen, and the virus is hepatitis C virus.

14. The method of claim 10, wherein said culturing is carried out in a serum-free medium which allows growth of the cell line without loss of liver-specific functions.

15. A method of screening compounds for ability to inhibit growth of an hepatotropic virus, comprising
    culturing the immortalized primate hepatocyte cell line of claim 1, after cell infection with the hepatotropic virus,
exposing the infected cells to the compound for a selected period, and
assaying the cells for inhibition of virus growth.

16. The method of claim 15, wherein said assaying includes detecting the level of virus-specific nucleic acid present in the cell culture.

17. The method of claim 16, wherein the cell line is derived from human or chimpanzee hepatocytes, and the virus is hepatitis C virus (HCV).

18. The method of claim 17, wherein the cells are infected with HCV by exposing the cells in culture to chimpanzee or human HCV-infected serum.

19. A method of producing a liver secretory protein selected from the group consisting of albumin, $\alpha$-1-antitrypsin, complement C'4, fibrinogen, apolipoproteins A-1 and E, transferrin, and plasminogen, comprising
culturing the cell line of claim 1 in a culture medium capable of maintaining the cells in the cell line in substantially differentiated state, and
isolating the protein from the culture medium.

20. The method of claim 19, wherein the protein is fibrinogen or plasminogen.

21. A method of producing the cell line according to any one of claims 1 to 9, the method comprising:
isolating primate hepatocytes and culturing the hepatocytes in serum-free medium to permit the hepatocytes to grow in culture in a liver-differentiated form as evidenced by the continued production and secretion in culture of liver-specific proteins;
integrating into the genome of cultured hepatocyte cells an oncogene effective to inhibit normal cell replication control; and
isolating immortalized primate hepatocyte cells.

## Patentansprüche

1. Unsterblich gemachte Primaten-Hepatozyten-Zellinie, deren Zellen gekennzeichnet sind durch:
(a) ein Onkogen, das in das zelluläre Genom integriert ist,
(b) Sekretion von mindestens drei sekretorischen Hepatozyten-Proteinen in Kultur, welche aus der aus Albumin, $\alpha$-1-Antitrypsin, Komplement C'4, Fibrinogen, Apolipoproteinen A-1 und E, Transferrin und Plasminogen bestehenden Gruppe ausgewählt sind, und
(c) die Fähigkeit die Replikation von humanen hepatotropen Viren zu unterstutzen.

2. Zellinie nach Anspruch 1, wobei der relative Anteil derartiger durch die Zellen synthetisierter sekretorischer Proteine ähnlich dem in Primatenserum ist.

3. Zellinie nach Anspruch 2, wobei die Zellen Albumin, $\alpha$-1-Antitrypsin, Apolipoprotein-E und Fibrinogen sezernieren.

4. Zellinie nach Anspruch 3, welche von primären Schimpansen-Hepatozyten abgeleitet ist, und wobei die Zellen der Zellinie mit Hepatitis-C-Virus infizierbar sind und dessen Replikation unterstutzen.

5. Zellinie nach Anspruch 1, welche von primären humanen- oder Schimpansen-Hepatozyten abgeleitet ist und wobei die Zellen der Zellinie mit Hepatitis-C-Virus infizierbar sind und dessen Replikation unterstutzen.

6. Zellinie nach Anspruch 1, welche mit Hepatitis B oder Hepatitis-A-Viren infizierbar ist und deren Replikation unterstützt.

7. Zellinie nach Anspruch 1, wobei das Onkogen aus der aus dem großen oder kleinen T-Antigen von SV-40, dem Adenovirus E1A, myc, ras oder Kombinationen davon bestehenden Gruppe ausgewählt ist.

8. Zellinie nach Anspruch 1, wobei die Zellinie von Schimpansen- oder primären humanen Hepatozyten abgeleitet ist und worin das Onkogen das große T-Antigen von SV-40 ist.

9. Zellinie nach Anspruch 1, worin die Zellinie von primären Pavian-Hepatozyten abgeleitet ist und worin das Onkogen eine Kombination von E1A und myc-Onkogen ist.

10. Verfahren zur Herstellung hepatotroper Viruspartikel, welches die folgenden Schritte umfaßt:
    Züchten der unsterblich gemachten Primaten-Hepatozyten-Zellinie nach Anspruch 1 nach Zellinfektion mit dem hepatotropen Virus und
    Gewinnen der Viruspartikel aus dem Kulturmedium.

11. Verfahren nach Anspruch 10, wobei die Zellinie von humanen oder Schimpansen-Hepatozyten abgeleitet ist und das Virus Hepatitis-C-Virus (HCV) ist.

12. Verfahren nach Anspruch 11, wobei die Zellen mit HCV infiziert werden, indem die Zellen in Kultur HCV infiziertem Schimpansen- oder humanem Serum ausgesetzt werden.

13. Verfahren nach Anspruch 10, wobei die Zellinie von Schimpansen- oder primären humanen Hepatozyten abgeleitet ist und das Onkogen das große T-Antigen von SV-40 ist und das Virus Hepatitis-C-Virus ist.

14. Verfahren nach Anspruch 10, wobei in einem serumfreien Medium, welches das Wachstum der Zellinie ohne Verlust leberspezifischer Funktionen ermöglicht, gezüchtet wird.

15. Verfahren zum Überprüfen von Verbindungen auf die Fähigkeit das Wachstum eines hepatotropen Virus zu inhibieren, welches darin besteht daß:
    die unsterblich gemachte Primaten-Hepatozyten-Zellinie nach Anspruch 1 nach Zellinfektion mit dem hepatotropen Virus gezüchtet wird,
    die infizierten Zellen für eine ausgewählte Zeitspanne der Verbindung ausgesetzt werden, und
    die Zellen auf Inhibierung des Viruswachstums geprüft werden.

16. Verfahren nach Anspruch 15, wobei die Prüfung den Nachweis der Menge an virusspezifischer Nucleinsäure, die in der Zellkultur vorhanden ist, einschließt.

17. Verfahren nach Anspruch 16, wobei die Zellinie von humanen oder Schimpansen-Hepatozyten abgeleitet ist und das Virus Hepatitis-C-Virus (HCV) ist.

18. Verfahren nach Anspruch 17, wobei die Zellen mit HCV infiziert werden, indem die Zellen in Kultur HCV-infiziertem Schimpansen- oder humanem Serum ausgesetzt werden.

19. Verfahren zur Herstellung eines sekretorischen Leber-Proteins, das aus der aus Albumin, $\alpha$-1-Antitrypsin, Komplement C'4, Fibrinogen, Apolipoproteinen A-1 und E, Transferrin und Plasminogen bestehenden Gruppe ausgewählt ist, welches darin besteht, daß:
    die Zellinie nach Anspruch 1 in einem Kulturmedium, in dem die Zellen der Zellinie in einem im wesentlichen differenzierten Stadium gehalten werden können, gezüchtet werden, und
    das Protein aus dem Kulturmedium isoliert wird.

20. Verfahren nach Anspruch 19, wobei das Protein Fibrinogen oder Plasminogen ist.

21. Verfahren zur Herstellung der Zellinie nach einem der Ansprüche 1 bis 9, welches darin besteht, daß:
    Primaten-Hepatozyten isoliert werden und die Hepatozyten in serumfreiem Medium gezüchtet werden, wobei den Hepatozyten das Wachstum in Kultur in einer Leber-differenzierten Form ermöglicht wird, wie durch die kontinuierliche Synthese und Sekretion von leberspezifischen Proteinen in Kultur nachgewiesen wird,
    ein Onkogen, das die normale Zellreplikationskontrolle wirksam inhibieren kann, in das Genom von gezüchteten Hepatozytenzellen inseriert wird, und
    die unsterblich gemachten Primaten-Hepatozyten-Zellen isoliert werden.

EP 0 527 814 B1

**Revendications**

1. Lignée cellulaire immortalisée d'hépatocytes de primate dont les cellules sont caractérisées par
   (a) un oncogène intégré dans le génome cellulaire ;
   (b) la sécrétion dans le milieu de culture d'au moins trois protéines sécréteuses d'hépatocytes choisies dans le groupe constitué de l'albumine, l'$\alpha$-1-antitrypsine, le complément C'4, le fibrinogène, les apolipoprotéines A-1 et E, la transferrine et le plasminogène ; et
   (c) la capacité à favoriser la réplication par les virus hépatotropes humains.

2. Lignée cellulaire de la revendication 1, dans laquelle la proportion relative de telles protéines de sécretion produites par les cellules est semblable à celle rencontrée dans le sérum de primates.

3. Lignée cellulaire de la revendication 2, dans laquelle les cellules secrètent de l'albumine, de l'$\alpha$-1-antitrypsine, de l'apolipoprotéine-E, et du fibrinogène.

4. Lignée cellulaire de la revendication 3, dérivée d'hépatocytes primaires de chimpanzés et dont les cellules, dans la lignée cellulaire, peuvent être infectées par le virus de l'hépatite C et favoriser la réplication de celui-ci.

5. Lignée cellulaire de la revendication 1, dérivée d'hépatocytes primaires humains ou de chimpanzés, dont les cellules, dans la lignée cellulaire, peuvent être infectées par le virus de l'hépatite C et favoriser la réplication de celui-ci.

6. Lignée cellulaire de la revendication 1, pouvant être infectée par les virus de l'hépatite B ou de l'hépatite A et favoriser leur réplication.

7. Lignée cellulaire de la revendication 1, dans laquelle l'oncogène est choisi dans le groupe constitué du petit ou grand antigène T de SV 40, de E1A de l'adéno virus, de myc, ras, et de combinaisons de ceux-ci.

8. Lignée cellulaire de la revendication 1, dans laquelle la lignée cellulaire dérive d'hépatocytes primaires humains ou de chimpanzés et l'oncogène est le grand antigène T de SV 40.

9. Lignée cellulaire de la revendication 1, dans laquelle la lignée cellulaire dérive d'hépatocytes primaires de babouins et l'oncogène est une combinaison d'oncogènes E1A et myc.

10. Procédé pour la production de particules virales hépatotropes comprenant
    la culture de la lignée cellulaire immortalisée d'hépatocytes de primates de la revendication 1, après infection des cellules par le virus hépatotrope, et
    la récolte des particules virales à partir du milieu de culture.

11. Procédé de la revendication 10, dans lequel la lignée cellulaire, dérivée d'hépatocytes humains ou de chimpanzés et le virus est le virus de l'hépatite C (HCV).

12. Procédé de la revendication 11, dans lequel les cellules sont infectées par HCV par exposition de cellules en culture à du sérum infecté par de l'HCV humain ou de chimpanzé.

13. Procédé de la revendication 10, dans lequel la lignée cellulaire, dérive d'hépatocytes primaires humains ou de chimpanzés, l'oncongène est le grand antigène T de SV 40 et le virus est le virus de l'hépatite C.

14. Procédé de la revendication 10, dans lequel ladite culture est réalisée dans un milieu dénué de sérum, permettant la croissance de la lignée cellulaire sans perte des fonctions spécifiques hépatiques.

15. Procédé pour le criblage de composés capables d'inhiber la croissance d'un virus hépatotrope, comprenant
    la culture de la lignée cellulaire immortalisée d'hépatocytes de primates de la revendication 1, après infection des cellules par le virus hépatotrope,
    l'exposition des cellules infectées au composé pendant une période déterminée, et

22

le dosage des cellules en terme d'inhibition de la croissance du virus.

16. Procédé de la revendication 15, dans lequel ledit dosage comprend une étape de détection du niveau d'acide nucléique spécifique du virus présent dans la culture cellulaire.

17. Procédé de la revendication 16, dans lequel la lignée cellulaire, dérive d'hépatocytes humains ou de chimpanzés et le virus est le virus de l'hépatite C (HCV).

18. Procédé de la revendication 17, dans lequel les cellules sont infectées par HCV par exposition des cellules en culture à du sérum infecté par de l'HCV humain ou de chimpanzé.

19. Procédé pour la production de protéines de sécrétion hépatiques choisies dans le groupe constitué de l'albumine, de l'$\alpha$-1-antitrypsine, du complément C'4, du fibrinogène, des apolipoprotéines A-1 et E, de la transferrine et du plasminogène comprenant
    la culture de la lignée cellulaire de la revendication 1 dans un milieu de culture capable de maintenir les cellules dans la lignée cellulaire dans un état essentiellement différencié et,
    l'isolement de la protéine à partir du milieu de culture.

20. Procédé de la revendication 19, dans lequel la protéine est le fibrinogène ou le plasminogène.

21. Procédé pour la production de la lignée cellulaire selon l'une quelconque des revendications 1 à 9, ledit procédé comprenant
    l'isolement d'hépatocytes de primates et la culture de ces hépatocytes dans un milieu dénué de sérum de façon à permettre la croissance des hépatocytes en culture dans un forme hépatique différenciée ainsi que mis en évidence par la production et la sécrétion continue de protéines hépatiques spécifiques dans le milieu de culture,
    l'intégration dans le génome de cellules d'hépatocytes en culture, d'un oncongène permettant l'inhibition de la fonction de régulation de la réplication des cellules normales ; et
    l'isolement des cellules immortalisées d'hépatocytes de primates.

FIG. IA

FIG. IB

FIG. IC

FIG. ID

EP 0 527 814 B1

FIG. 2B

FIG. 2D

FIG. 2A

FIG. 2C

FIG. 6

FIG. 3

FIG. 4A

FIG. 4B

FIG. 7

FIG. 5

1 2 3 4 5 6 7 8 9 10 11 12 13

FIG. 8